# EUROPEAN PATENT SPECIFICATION

(11) **EP 2 968 460 B1**
(45) Date of publication and mention of the grant of the patent: **06.01.2021**
(21) Application number: 14721569.3
(22) Date of filing: 07.03.2014
(51) Int. Cl.: A61K 39/395, A61K 38/18, A61K 9/19, A61K 47/42, A61K 47/26, A61K 47/02, C07K 16/22

(54) **PROTEIN FORMULATIONS**
PROTEINFORMULIERUNGEN
FORMULATIONS PROTÉIQUES

(30) Priority: 11.03.2013 US 201361775974 P
(43) Date of publication of application: 20.01.2016
(73) Proprietor: Amgen Inc., Thousand Oaks, California 91320-1799 (US)
(72) Inventor: PARK, Sungae, Thousand Oaks, California 91320 (US); KUHNS, Scott, Newbury Park, California 91320 (US); RATTAN, Jennifer, Thousand Oaks, California 91360 (US); PHILLIPS, Joseph, Newbury Park, California 91320 (US)
(74) Representative: Lee, Nicholas John
(86) International application number: PCT/US2014/021792
(87) International publication number: WO 2014/164301

(56) References cited:
- EP-A2- 0 178 665
- US-A1- 2012 076 800
- US-B1- 6 267 958
- WEI WANG: "Lyophilization and development of solid protein pharmaceuticals", INTERNATIONAL JOURNAL OF PHARMACEUTICS, vol. 203, no. 1-2, 1 August 2000 (2000-08-01), pages 1-60, XP055005330, ISSN: 0378-5173, DOI: 10.1016/S0378-5173(00)00423-3

## Description

This application claims the benefit of U.S. Provisional Patent Application No. 61/775,974 filed March 11, 2013.

### FIELD OF THE INVENTION

The field of this invention relates to compositions and methods related to protein formulations.

### BACKGROUND OF THE INVENTION

Erythropoietin (EPO) is a glycoprotein hormone necessary for the maturation of erythroid progenitor cells into erythrocytes. It is produced in the kidney and is essential in regulating levels of red blood cells in the circulation. Conditions marked by low levels of tissue oxygen signal increased production of EPO, which in turn stimulates erythropoiesis. A loss of kidney function as is seen in chronic renal failure, for example, typically results in decreased production of EPO and a concomitant reduction in red blood cells.

Human urinary EPO was purified by Miyake et al. (J. Biol. Chem. 252, 5558 (1977)) from patients with aplastic anemia. However, the amount of purified EPO protein obtained from this source was insufficient for therapeutic applications. The identification and cloning of the gene encoding human EPO and expression of recombinant protein was disclosed in U.S. Pat. No. 4,703,008 to Lin. A method for purification of recombinant human erythropoietin from cell medium is disclosed in U.S. Pat. No. 4,667,016 to Lai et. al. The production of biologically active EPO from mammalian host cells made available, for the first time, quantities of EPO suitable for therapeutic applications. In addition, knowledge of the gene sequence and the increased availability of purified protein led to a better understanding of the mode of action of this protein. US6267958B1 describes a stable lyophilized protein formulation which can be reconstituted with a suitable diluent to generate a high protein concentration reconstituted formulation which is suitable for subcutaneous administration.

As is often the case with a successful, marketed product, counterfeit products are put into the stream of commerce in an attempt to unscrupulously profit off of the name of the original product. In the pharmaceutical industry, this can be quite dangerous, as a patient in need of the drug will not be receiving the expected therapeutic dose if a counterfeit drug is administered. It is imperative that counterfeit drugs be detected before they enter the stream of commerce and end up being administered to unknowing patients. Accordingly, there is a need for stable protein formulations that can be used in sensitive, reproducible assays.

### SUMMARY OF THE INVENTION

In one embodiment, the invention provides a stable, solid protein formulation comprising bovine serum albumin (BSA), potassium phosphate, mannitol, sucrose, and polysorbate 20, wherein said protein is darbepoetin. The concentration of BSA may be between 0.1% and 10%. The concentration of potassium phosphate may be between 1mM and 100mM. The concentration of mannitol may be between 1% and 10%. The concentration of sucrose may be between 1% and 10%. The concentration of polysorbate 20 may be between 0.005% and 0.5%. The pH may be between 6.0 and 8.0. The concentration of protein may be between 1 ng/ml and 100 ng/ml. The formulation may be lyophilized. The formulation may be reconstituted with water.

In another embodiment, the invention provides a lyophilization buffer comprising bovine serum albumin (BSA), potassium phosphate, mannitol, sucrose, and polysorbate 20, optionally wherein the buffer further comprises darbepoetin. The concentration of BSA may be between 0.1% and 10%. The concentration of potassium phosphate may be between 1mM and 100mM. The concentration of mannitol may be between 1% and 10%. The concentration of sucrose may be between 1% and 10%. The concentration of polysorbate 20 may be between 0.005% and 0.5%. The pH may be between 6.0 and 8.0. The concentration of protein may be between 1 ng/ml and 100 ng/ml. The buffer may be lyophilized. The buffer may be reconstituted with water.

In a further embodiment, the invention provides a method of preparing a stable, solid protein formulation, said method comprising: a) diluting a protein with the lyophilization buffer of the invention; and b) lyophilizing the diluted protein.

Disclosed herein is a kit comprising a stable, solid erythropoietin protein formulation and a stable, solid anti-erythropoietin antibody formulation.

Disclosed herein is a method of testing a sample for the presence of erythropoietin, said method comprising: a) reconstituting a stable, solid erythropoietin formulation and a stable, solid anti-erythropoietin antibody formulation with water; b) contacting the anti-erythropoietin antibody formulation of a) with a test sample and assaying for binding, wherein a positive binding result indicates the presence of erythropoietin.

### BRIEF DESCRIPTION OF THE FIGURES

**Figure 1.** Figure 1 describes an erythropoietin detection ELISA assay that compares several different anti-erythropoietin antibodies.

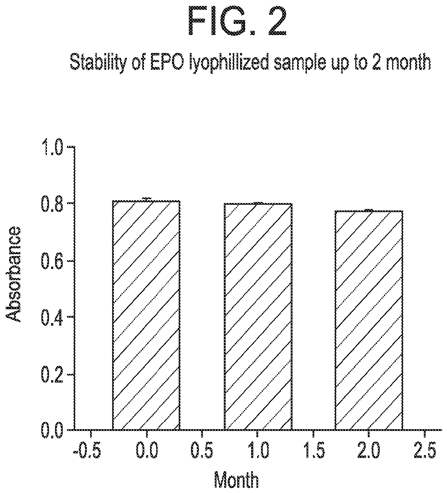

**Figure 2.** Figure 2 demonstrates the stability of the lyophilized erythropoietin sample at 0, 1 and 2 month timepoints.

### DETAILED DESCRIPTION OF THE INVENTION

The present invention relates to stable, solid protein formulations. The present invention further provides compositions and methods relating to stable, solid protein formulations. The present invention further relates to compositions and methods for preparing said compositions. Methods for detecting the presence or absence of recombinant proteins, for example, but not limited to, recombinant human erythropoietin are disclosed. Kits relating to stable, solid protein formulations are disclosed.

### Definitions

Unless otherwise defined herein, scientific and technical terms used in connection with the present invention shall have the meanings that are commonly understood by those of ordinary skill in the art. Further, unless otherwise required by context, singular terms shall include pluralities and plural terms shall include the singular. Generally, nomenclatures used in connection with, and techniques of, cell and tissue culture, molecular biology, immunology, microbiology, genetics and protein and nucleic acid chemistry and hybridization described herein are those well known and commonly used in the art. The methods and techniques of the present invention are generally performed according to conventional methods well known in the art and as described in various general and more specific references that are cited and discussed throughout the present specification unless otherwise indicated. See, e.g., Sambrook et al. Molecular Cloning: A Laboratory Manual, 2d ed., Cold Spring Harbor Laboratory Press, Cold Spring Harbor, N.Y. (1989) and Ausubel et al., Current Protocols in Molecular Biology, Greene Publishing Associates (1992), and Harlow and Lane Antibodies: A Laboratory Manual Cold Spring Harbor Laboratory Press, Cold Spring Harbor, N.Y. (1990). Enzymatic reactions and purification techniques are performed according to manufacturer's specifications, as commonly accomplished in the art or as described herein. The terminology used in connection with, and the laboratory procedures and techniques of, analytical chemistry, synthetic organic chemistry, and medicinal and pharmaceutical chemistry described herein are those well known and commonly used in the art. Standard techniques can be used for chemical syntheses, chemical analyses, pharmaceutical preparation, formulation, and delivery, and treatment of patients.

The following terms, unless otherwise indicated, shall be understood to have the following meanings:
A "glycoprotein" is a protein that has covalently attached oligosaccharides to polypeptide side chains.

An "immunoglobulin" is a tetrameric molecule. In a naturally occurring immunoglobulin, each tetramer is composed of two identical pairs of polypeptide chains, each pair having one "light" (about 25 kDa) and one "heavy" chain (about 50-70 kDa). The amino-terminal portion of each chain includes a variable region of about 100 to 110 or more amino acids primarily responsible for antigen recognition. The carboxy-terminal portion of each chain defines a constant region primarily responsible for effector function. Human light chains are classified as kappa and lambda light chains. Heavy chains are classified as mu, delta, gamma, alpha, or epsilon, and define the antibody's isotype as IgM, IgD, IgG, IgA, and IgE, respectively. Within light and heavy chains, the variable and constant regions are joined by a "J" region of about 12 or more amino acids, with the heavy chain also including a "D" region of about 10 more amino acids. See generally, Fundamental Immunology Ch. 7 (Paul, W., ed., 2nd ed. Raven Press, N.Y. (1989)). The variable regions of each light/heavy chain pair form the antibody binding site such that an intact immunoglobulin has two binding sites.

Naturally occurring immunoglobulin chains exhibit the same general structure of relatively conserved framework regions (FR) joined by three hypervariable regions, also called complementarity determining regions or CDRs. From N-terminus to C-terminus, both light and heavy chains comprise the domains FR1, CDR1, FR2, CDR2, FR3, CDR3 and FR4. The assignment of amino acids to each domain is in accordance with the definitions of Kabat et al. in Sequences of Proteins of Immunological Interest, 5th Ed., US Dept. of Health and Human Services, PHS, NIH, NIH Publication no. 91-3242, 1991.

An "antibody" refers to an intact immunoglobulin or to an antigen binding portion thereof that competes with the intact antibody for specific binding, unless otherwise specified. Antigen binding portions may be produced by recombinant DNA techniques or by enzymatic or chemical cleavage of intact antibodies. Antigen binding portions include, inter alia, Fab, Fab', F(ab')₂, Fv, domain antibodies (dAbs), fragments including complementarity determining regions (CDRs), single-chain antibodies (scFv), chimeric antibodies, diabodies, triabodies, tetrabodies, and polypeptides that contain at least a portion of an immunoglobulin that is sufficient to confer specific antigen binding to the polypeptide.

A Fab fragment is a monovalent fragment having the V_{L}, V_{H}, C_{L} and C_{H}1 domains; a F(ab')₂ fragment is a bivalent fragment having two Fab fragments linked by a disulfide bridge at the hinge region; a Fd fragment has the V_{H} and C_{H}1 domains; an Fv fragment has the V_{L} and V_{H} domains of a single arm of an antibody; and a dAb fragment has a V_{H} domain, a V_{L} domain, or an antigen-binding fragment of a V_{H} or V_{L} domain (US Pat. No. 6,846,634, 6,696,245, US App. Pub. No. 05/0202512, 04/0202995, 04/0038291, 04/0009507, 03/0039958, Ward et al., Nature 341:544-546 (1989)).

A single-chain antibody (scFv) is an antibody in which a V_{L} and a V_{H} region are joined via a linker (e.g., a synthetic sequence of amino acid residues) to form a continuous protein chain wherein the linker is long enough to allow the protein chain to fold back on itself and form a monovalent antigen binding site (see, e.g., Bird et al., Science 242:423-26 (1988) and Huston et al., 1988, Proc. Natl. Acad. Sci. USA 85:5879-83 (1988)). Diabodies are bivalent antibodies comprising two polypeptide chains, wherein each polypeptide chain comprises V_{H} and V_{L} domains joined by a linker that is too short to allow for pairing between two domains on the same chain, thus allowing each domain to pair with a complementary domain on another polypeptide chain (see, e.g., Holliger et al., 1993, Proc. Natl. Acad. Sci. USA 90:6444-48 (1993), and Poljak et al., Structure 2:1121-23 (1994)). If the two polypeptide chains of a diabody are identical, then a diabody resulting from their pairing will have two identical antigen binding sites. Polypeptide chains having different sequences can be used to make a diabody with two different antigen binding sites. Similarly, tribodies and tetrabodies are antibodies comprising three and four polypeptide chains, respectively, and forming three and four antigen binding sites, respectively, which can be the same or different.

Complementarity determining regions (CDRs) and framework regions (FR) of a given antibody may be identified using the system described by Kabat et al. in Sequences of Proteins of Immunological Interest, 5th Ed., US Dept. of Health and Human Services, PHS, NIH, NIH Publication no. 91-3242, 1991. One or more CDRs may be incorporated into a molecule either covalently or noncovalently to make it an antibody. An antibody may incorporate the CDR(s) as part of a larger polypeptide chain, may covalently link the CDR(s) to another polypeptide chain, or may incorporate the CDR(s) noncovalently. The CDRs permit the antibody to specifically bind to a particular antigen of interest. Disclosed is a binding molecule that comprises at least one, at least two, at least three, at least four, at least five, or six CDRs from the antibodies of the invention.

Fragments or analogs of antibodies can be readily prepared by those of ordinary skill in the art following the teachings of this specification and using techniques well-known in the art. Preferred amino- and carboxy-termini of fragments or analogs occur near boundaries of functional domains.

### Formulations

The present invention relates to stable, solid protein formulations. Given that protein detection kits to be stored and used in a variety of environmental conditions (e.g., wide temperature ranges) are disclosed, the invention provides such formulations that maintain protein stability in a variety of these environmental conditions. Disclosed herein are test kits and solutions that can be used under a wide variety of environmental conditions to assay for the presence of recombinant protein (e.g., a therapeutic protein such as recombinant human erythropoietin). With the environmental conditions that the test kits and solutions will be subjected to, it is desired and contemplated that such kits and solutions provide long term stability at various temperature ranges so that activity of the kits and solutions remains present.

In one embodiment, the invention provides a stable, solid protein formulation comprising bovine serum albumin (BSA), potassium phosphate, mannitol, sucrose, and polysorbate 20.

In another embodiment, the invention provides a lyophilization buffer comprising bovine serum albumin (BSA), potassium phosphate, mannitol, sucrose, and polysorbate 20.

Given the inventive combination of bovine serum albumin (BSA), potassium phosphate, mannitol, sucrose, and polysorbate 20, one can determine appropriate concentrations of each excipient to achieve the desired stability of the solid protein formulation. This stability can be tested using assays described herein, or with additional assays known in the art.

For example, in one embodiment, the concentration of BSA is between about 0.1% and about 10%. In a further embodiment, the concentration of BSA is between about 0.1% and about 1%. In another embodiment, the concentration of BSA is between about 0.1% and about 5%. In another embodiment, the concentration of BSA is between about 1% and about 10%. In another embodiment, the concentration of BSA is between about 1% and about 5%. In another embodiment, the concentration of BSA is between about 0.5% and about 2%. In another embodiment, the concentration of BSA is about 2%. In another embodiment, the concentration of BSA is about 3%. In another embodiment, the concentration of BSA is about 4%. In another embodiment, the concentration of BSA is about 5%. In another embodiment, the concentration of BSA is about 0.5%. In yet another embodiment, the concentration of BSA is about 1%. In one embodiment, the concentration of BSA is between 0.1% and 10%. In a further embodiment, the concentration of BSA is between 0.1% and 1%. In another embodiment, the concentration of BSA is between 0.1% and 5%. In another embodiment, the concentration of BSA is between 1% and 10%. In another embodiment, the concentration of BSA is between 1% and 5%. In another embodiment, the concentration of BSA is between 0.5% and 2%. In another embodiment, the concentration of BSA is 2%. In another embodiment, the concentration of BSA is 3%. In another embodiment, the concentration of BSA is 4%. In another embodiment, the concentration of BSA is 5%. In another embodiment, the concentration of BSA is 0.5%. In yet another embodiment, the concentration of BSA is 1%.

In one embodiment, the concentration of potassium phosphate is between about 1mM and about 100mM. In another embodiment, the concentration of potassium phosphate is between about 1mM and about 50mM. In another embodiment, the concentration of potassium phosphate is between about 1mM and about 25mM. In another embodiment, the concentration of potassium phosphate is between about 1mM and about 10mM. In another embodiment, the concentration of potassium phosphate is between about 5mM and about 50mM. In another embodiment, the concentration of potassium phosphate is between about 5mM and about 25mM. In another embodiment, the concentration of potassium phosphate is between about 5mM and about 10mM. In another embodiment, the concentration of potassium phosphate is between about 10mM and about 100mM. In another embodiment, the concentration of potassium phosphate is between about 10mM and about 50mM. In another embodiment, the concentration of potassium phosphate is between about 10mM and about 25mM. In another embodiment, the concentration of potassium phosphate is about 1mM. In another embodiment, the concentration of potassium phosphate is about 5mM. In another embodiment, the concentration of potassium phosphate is about 10mM. In another embodiment, the concentration of potassium phosphate is about 15mM. In another embodiment, the concentration of potassium phosphate is about 20mM. In another embodiment, the concentration of potassium phosphate is about 25mM. In another embodiment, the concentration of potassium phosphate is about 50mM. In another embodiment, the concentration of potassium phosphate is about 75mM. In another embodiment, the concentration of potassium phosphate is about 100mM.

In one embodiment, the concentration of potassium phosphate is between 1mM and 100mM. In another embodiment, the concentration of potassium phosphate is between 1mM and 50mM. In another embodiment, the concentration of potassium phosphate is between 1mM and 25mM. In another embodiment, the concentration of potassium phosphate is between 1mM and 10mM. In another embodiment, the concentration of potassium phosphate is between 5mM and 50mM. In another embodiment, the concentration of potassium phosphate is between 5mM and 25mM. In another embodiment, the concentration of potassium phosphate is between 5mM and 10mM. In another embodiment, the concentration of potassium phosphate is between 10mM and 100mM. In another embodiment, the concentration of potassium phosphate is between 10mM and 50mM. In another embodiment, the concentration of potassium phosphate is between 10mM and 25mM. In another embodiment, the concentration of potassium phosphate is 1mM. In another embodiment, the concentration of potassium phosphate is 5mM. In another embodiment, the concentration of potassium phosphate is 10mM. In another embodiment, the concentration of potassium phosphate is 15mM. In another embodiment, the concentration of potassium phosphate is 20mM. In another embodiment, the concentration of potassium phosphate is 25mM. In another embodiment, the concentration of potassium phosphate is 50mM. In another embodiment, the concentration of potassium phosphate is 75mM. In another embodiment, the concentration of potassium phosphate is 100mM.

In one embodiment, the concentration of mannitol is between about 1% and about 10%. In another embodiment, the concentration of mannitol is between about 1% and about 5%. In another embodiment, the concentration of mannitol is between about 1% and about 3%. In another embodiment, the concentration of mannitol is between about 3% and about 5%. In another embodiment, the concentration of mannitol is between about 5% and about 10%. In another embodiment, the concentration of mannitol is about 1%. In another embodiment, the concentration of mannitol is about 2%. In another embodiment, the concentration of mannitol is about 3%. In another embodiment, the concentration of mannitol is about 4%. In another embodiment, the concentration of mannitol is about 5%. In another embodiment, the concentration of mannitol is about 6%. In another embodiment, the concentration of mannitol is about 7%. In another embodiment, the concentration of mannitol is about 8%. In another embodiment, the concentration of mannitol is about 9%. In another embodiment, the concentration of mannitol is about 10%.

In one embodiment, the concentration of mannitol is between 1% and 10%. In another embodiment, the concentration of mannitol is between 1% and 5%. In another embodiment, the concentration of mannitol is between 1% and 3%. In another embodiment, the concentration of mannitol is between 3% and 5%. In another embodiment, the concentration of mannitol is between 5% and 10%. In another embodiment, the concentration of mannitol is 1%. In another embodiment, the concentration of mannitol is 2%. In another embodiment, the concentration of mannitol is 3%. In another embodiment, the concentration of mannitol is 4%. In another embodiment, the concentration of mannitol is 5%. In another embodiment, the concentration of mannitol is 6%. In another embodiment, the concentration of mannitol is 7%. In another embodiment, the concentration of mannitol is 8%. In another embodiment, the concentration of mannitol is 9%. In another embodiment, the concentration of mannitol is 10%.

In one embodiment, the concentration of sucrose is between about 1% and about 10%. In another embodiment, the concentration of sucrose is between about 1% and about 5%. In another embodiment, the concentration of sucrose is between about 1% and about 3%. In another embodiment, the concentration of sucrose is between about 3% and about 5%. In another embodiment, the concentration of sucrose is between about 5% and about 10%. In another embodiment, the concentration of sucrose is about 1%. In another embodiment, the concentration of sucrose is about 2%. In another embodiment, the concentration of sucrose is about 3%. In another embodiment, the concentration of sucrose is about 4%. In another embodiment, the concentration of sucrose is about 5%. In another embodiment, the concentration of sucrose is about 6%. In another embodiment, the concentration of sucrose is about 7%. In another embodiment, the concentration of sucrose is about 8%. In another embodiment, the concentration of sucrose is about 9%. In another embodiment, the concentration of sucrose is about 10%.

In one embodiment, the concentration of sucrose is between 1% and 10%. In another embodiment, the concentration of sucrose is between 1% and 5%. In another embodiment, the concentration of sucrose is between 1% and 3%. In another embodiment, the concentration of sucrose is between 3% and 5%. In another embodiment, the concentration of sucrose is between 5% and 10%. In another embodiment, the concentration of sucrose is 1%. In another embodiment, the concentration of sucrose is 2%. In another embodiment, the concentration of sucrose is 3%. In another embodiment, the concentration of sucrose is 4%. In another embodiment, the concentration of sucrose is 5%. In another embodiment, the concentration of sucrose is 6%. In another embodiment, the concentration of sucrose is 7%. In another embodiment, the concentration of sucrose is 8%. In another embodiment, the concentration of sucrose is 9%. In another embodiment, the concentration of sucrose is 10%.

In one embodiment, the concentration of polysorbate 20 is between about 0.005% and about 0.5%. In another embodiment, the concentration of polysorbate 20 is between about 0.005% and about 0.1%. In another embodiment, the concentration of polysorbate 20 is between about 0.005% and about 0.05%. In another embodiment, the concentration of polysorbate 20 is between about 0.01% and about 0.1%. In another embodiment, the concentration of polysorbate 20 is between about 0.5% and about 0.1%. In another embodiment, the concentration of polysorbate 20 is about 0.005%. In another embodiment, the concentration of polysorbate 20 is about 0.001%. In another embodiment, the concentration of polysorbate 20 is about 0.05%. In another embodiment, the concentration of polysorbate 20 is about 0.01%. In another embodiment, the concentration of polysorbate 20 is about 0.05%. In another embodiment, the concentration of polysorbate 20 is about 0.1%. In another embodiment, the concentration of polysorbate 20 is about 0.5%.

In one embodiment, the concentration of polysorbate 20 is between 0.005% and 0.5%. In another embodiment, the concentration of polysorbate 20 is between 0.005% and 0.1%. In another embodiment, the concentration of polysorbate 20 is between 0.005% and 0.05%. In another embodiment, the concentration of polysorbate 20 is between 0.01% and 0.1%. In another embodiment, the concentration of polysorbate 20 is between 0.5% and 0.1%. In another embodiment, the concentration of polysorbate 20 is 0.005%. In another embodiment, the concentration of polysorbate 20 is 0.001%. In another embodiment, the concentration of polysorbate 20 is 0.05%. In another embodiment, the concentration of polysorbate 20 is 0.01%. In another embodiment, the concentration of polysorbate 20 is 0.05%. In another embodiment, the concentration of polysorbate 20 is 0.1%. In another embodiment, the concentration of polysorbate 20 is 0.5%.

In one embodiment, the pH is between about 6.0 and about 8.0. In another embodiment, the pH is between about 4.0 and about 10.0. In another embodiment, the pH is between about 5.0 and about 7.0. In another embodiment, the pH is between about 5.0 and about 9.0. In another embodiment, the pH is between about 6.0 and about 9.0. In another embodiment, the pH is about 5.0. In another embodiment, the pH is about 6.0. In another embodiment, the pH is about 6.5. In another embodiment, the pH is about 7.0. In another embodiment, the pH is about 7.5. In another embodiment, the pH is about 8.0. In another embodiment, the pH is about 8.5. In another embodiment, the pH is about 9.0. In another embodiment, the pH is about 9.5. In another embodiment, the pH is about 10.0.

In one embodiment, the pH is between 6.0 and 8.0. In another embodiment, the pH is between 4.0 and 10.0. In another embodiment, the pH is between 5.0 and 7.0. In another embodiment, the pH is between 5.0 and 9.0. In another embodiment, the pH is between 6.0 and 9.0. In another embodiment, the pH is 5.0. In another embodiment, the pH is 6.0. In another embodiment, the pH is 6.5. In another embodiment, the pH is 7.0. In another embodiment, the pH is 7.5. In another embodiment, the pH is 8.0. In another embodiment, the pH is 8.5. In another embodiment, the pH is 9.0. In another embodiment, the pH is 9.5. In another embodiment, the pH is 10.0.

In one embodiment, the concentration of protein is between about 1 ng/ml and about 100 ng/ml. In another embodiment, the concentration of protein is between about 1 ng/ml and about 50 ng/ml. In another embodiment, the concentration of protein is between about 1 ng/ml and about 25 ng/ml. In another embodiment, the concentration of protein is between about 1 ng/ml and about 10 ng/ml. In another embodiment, the concentration of protein is between about 5 ng/ml and about 50 ng/ml. In another embodiment, the concentration of protein is between about 5 ng/ml and about 25 ng/ml. In another embodiment, the concentration of protein is between about 10 ng/ml and about 100 ng/ml. In another embodiment, the concentration of protein is between about 10 ng/ml and about 50 ng/ml. In another embodiment, the concentration of protein is between about 10 ng/ml and about 25 ng/ml. In another embodiment, the concentration of protein is about 1 ng/ml. In another embodiment, the concentration of protein is about 5 ng/ml. In another embodiment, the concentration of protein is about 10 ng/ml. In another embodiment, the concentration of protein is about 20 ng/ml. In another embodiment, the concentration of protein is about 30 ng/ml. In another embodiment, the concentration of protein is about 40 ng/ml. In another embodiment, the concentration of protein is about 50 ng/ml. In another embodiment, the concentration of protein is about 60 ng/ml. In another embodiment, the concentration of protein is about 70 ng/ml. In another embodiment, the concentration of protein is about 80 ng/ml. In another embodiment, the concentration of protein is about 90 ng/ml. In another embodiment, the concentration of protein is about 100 ng/ml.

In one embodiment, the concentration of protein is between 1 ng/ml and 100 ng/ml. In another embodiment, the concentration of protein is between 1 ng/ml and 50 ng/ml. In another embodiment, the concentration of protein is between 1 ng/ml and 25 ng/ml. In another embodiment, the concentration of protein is between 1 ng/ml and 10 ng/ml. In another embodiment, the concentration of protein is between 5 ng/ml and 50 ng/ml. In another embodiment, the concentration of protein is between 5 ng/ml and 25 ng/ml. In another embodiment, the concentration of protein is between 10 ng/ml and 100 ng/ml. In another embodiment, the concentration of protein is between 10 ng/ml and 50 ng/ml. In another embodiment, the concentration of protein is between 10 ng/ml and 25 ng/ml. In another embodiment, the concentration of protein is 1 ng/ml. In another embodiment, the concentration of protein is 5 ng/ml. In another embodiment, the concentration of protein is 10 ng/ml. In another embodiment, the concentration of protein is 20 ng/ml. In another embodiment, the concentration of protein is 30 ng/ml. In another embodiment, the concentration of protein is 40 ng/ml. In another embodiment, the concentration of protein is 50 ng/ml. In another embodiment, the concentration of protein is 60 ng/ml. In another embodiment, the concentration of protein is 70 ng/ml. In another embodiment, the concentration of protein is 80 ng/ml. In another embodiment, the concentration of protein is 90 ng/ml. In another embodiment, the concentration of protein is 100 ng/ml.

In a specific embodiment, the invention provides a stable, solid protein formulation comprising bovine serum albumin (BSA), potassium phosphate, mannitol, sucrose, and polysorbate 20, wherein said protein concentration is about 10ng/ml, said BSA concentration is about 1%, said potassium phosphate concentration is about 10mM, said mannitol concentration is about 4%, said sucrose concentration is about 2%, and said polysorbate 20 concentration is about 0.05%. In a further specific embodiment, the pH of the aforementioned protein formulation is about 7.0.

In a further embodiment, the stable, solid protein formulation is lyophilized. Lyophilization, also known as freeze-drying, is a widely used dehydration process used to preserve material (e.g., proteins), where the material is rapidly frozen and dehydrated while under vacuum conditions.

In another embodiment, the invention provides a method of preparing a stable, solid protein formulation, said method comprising: a) diluting said protein with the lyophilization buffer of the invention; and b) lyophilizing said diluted protein.

Disclosed herein is a kit comprising a stable, solid erythropoietin protein formulation and a stable, solid anti-erythropoietin antibody formulation. All reagents of the kit may be room temperature stable. All reagents of the kit may be stable in a wide variety of temperature ranges as previously described herein. The kit may comprise an anti-erythropoietin horseradish peroxidase (HRP) conjugated antibody (e.g. Sigma Cat# P8375-250 KU); recombinant human erythropoietin (e.g., Epogen®); bovine serum polysorbate (BSAT) solution; wash buffer; color developer; and erythropoietin dilution solution.

Disclosed herein is a method of testing a sample for the presence of erythropoietin, said method comprising: a) reconstituting a stable, solid erythropoietin formulation and a stable, solid anti-erythropoietin antibody formulation with water; b) contacting the anti-erythropoietin antibody formulation of a) with a test sample and assaying for binding, wherein a positive binding result indicates the presence of erythropoietin.

In a specific embodiment of the invention, the formulation of the invention does not comprise histidine.

In one embodiment, the stable, solid protein formulation is stable for at least 30 days at 25 degrees C, 40 degrees C, and 45 degrees C. In another embodiment, the stable, solid protein formulation is stable for at least 60 days at 25 degrees C, 40 degrees C, and 45 degrees C. In another embodiment, the stable, solid protein formulation is stable for at least 90 days at 25 degrees C, 40 degrees C, and 45 degrees C. In another embodiment, the stable, solid protein formulation is stable for at least 120 days at 25 degrees C, 40 degrees C, and 45 degrees C. In another embodiment, the stable, solid protein formulation is stable for at least 6 months at 25 degrees C, 40 degrees C, and 45 degrees C. In another embodiment, the stable, solid protein formulation is stable for at least 9 months at 25 degrees C, 40 degrees C, and 45 degrees C. In another embodiment, the stable, solid protein formulation is stable for at least 12 months at 25 degrees C, 40 degrees C, and 45 degrees C. In another embodiment, the stable, solid protein formulation is stable for at least 18 months at 25 degrees C, 40 degrees C, and 45 degrees C. In another embodiment, the stable, solid protein formulation is stable for at least 24 months at 25 degrees C, 40 degrees C, and 45 degrees C. In another embodiment, the stable, solid protein formulation is stable for at least 36 months at 25 degrees C, 40 degrees C, and 45 degrees C. In another embodiment, the stable, solid protein formulation is stable for at least 48 months at 25 degrees C, 40 degrees C, and 45 degrees C. In another embodiment, the stable, solid protein formulation is stable for at least 60 months at 25 degrees C, 40 degrees C, and 45 degrees C.

In one embodiment, the stable, solid protein formulation is stable for at least 30 days at 25 degrees C. In one embodiment, the stable, solid protein formulation is stable for at least 30 days at 40 degrees C. In one embodiment, the stable, solid protein formulation is stable for at least 30 days at 45 degrees C.

In another embodiment, the stable, solid protein formulation is stable for at least 60 days at 25 degrees C. In another embodiment, the stable, solid protein formulation is stable for at least 60 days at 40 degrees C. In another embodiment, the stable, solid protein formulation is stable for at least 60 days at 45 degrees C.

In another embodiment, the stable, solid protein formulation is stable for at least 90 days at 25 degrees C. In another embodiment, the stable, solid protein formulation is stable for at least 90 days at 40 degrees C. In another embodiment, the stable, solid protein formulation is stable for at least 90 days at 45 degrees C.

In another embodiment, the stable, solid protein formulation is stable for at least 120 days at 25 degrees C. In another embodiment, the stable, solid protein formulation is stable for at least 120 days at 40 degrees C. In another embodiment, the stable, solid protein formulation is stable for at least 120 days at 45 degrees C.

In another embodiment, the stable, solid protein formulation is stable for at least 6 months at 25 degrees C. In another embodiment, the stable, solid protein formulation is stable for at least 6 months at 40 degrees C. In another embodiment, the stable, solid protein formulation is stable for at least 6 months at 45 degrees C.

In another embodiment, the stable, solid protein formulation is stable for at least 9 months at 25 degrees C. In another embodiment, the stable, solid protein formulation is stable for at least 9 months at 40 degrees C. In another embodiment, the stable, solid protein formulation is stable for at least 9 months at 45 degrees C.

In another embodiment, the stable, solid protein formulation is stable for at least 12 months at 25 degrees C. In another embodiment, the stable, solid protein formulation is stable for at least 12 months at 40 degrees C. In another embodiment, the stable, solid protein formulation is stable for at least 12 months at 45 degrees C.

In another embodiment, the stable, solid protein formulation is stable for at least 18 months at 25 degrees C. In another embodiment, the stable, solid protein formulation is stable for at least 18 months at 40 degrees C. In another embodiment, the stable, solid protein formulation is stable for at least 18 months at 45 degrees C.

In another embodiment, the stable, solid protein formulation is stable for at least 24 months at 25 degrees C. In another embodiment, the stable, solid protein formulation is stable for at least 24 months at 40 degrees C. In another embodiment, the stable, solid protein formulation is stable for at least 24 months at 45 degrees C.

In another embodiment, the stable, solid protein formulation is stable for at least 36 months at 25 degrees C. In another embodiment, the stable, solid protein formulation is stable for at least 36 months at 40 degrees C. In another embodiment, the stable, solid protein formulation is stable for at least 36 months at 45 degrees C.

In another embodiment, the stable, solid protein formulation is stable for at least 48 months at 25 degrees C. In another embodiment, the stable, solid protein formulation is stable for at least 48 months at 40 degrees C. In another embodiment, the stable, solid protein formulation is stable for at least 48 months at 45 degrees C.

In another embodiment, the stable, solid protein formulation is stable for at least 60 months at 25 degrees C. In another embodiment, the stable, solid protein formulation is stable for at least 60 months at 40 degrees C. In another embodiment, the stable, solid protein formulation is stable for at least 60 months at 45 degrees C.

In certain embodiments, the protein used with the formulations and lyophilization buffers of the invention is a glycoprotein. The protein may be erythropoietin. In another embodiment, the protein is an analog of erythropoietin. In a further embodiment, the protein is darbepoetin alfa. The protein may be an antibody. The protein may be a monoclonal antibody or a polyclonal antibody.

### Erythropoietin

Erythropoietin is a glycoprotein hormone involved in the maturation of erythroid progenitor cells into erythrocytes. It is essential in regulating levels of red blood cells in circulation. Naturally occurring erythropoietin is produced by the liver during fetal life and by the kidney of adults and circulates in the blood and stimulates the production of red blood cells in bone marrow. Anemia is almost invariably a consequence of renal failure due to decreased production of erythropoietin from the kidney. Recombinant erythropoietin produced by genetic engineering techniques involving the expression of a protein product from a host cell transformed with the gene encoding erythropoietin has been found to be effective when used in the treatment of anemia resulting from chronic renal failure.

The identification, cloning, and expression of genes encoding erythropoietin are described in U.S. Pat. No. 4,703,008 to Lin. A description of the purification of recombinant erythropoietin from cell medium that supported the growth of mammalian cells containing recombinant erythropoietin plasmids for example, is included in U.S. Pat. No. 4,667,016 to Lai et al. The expression and recovery of biologically active recombinant erythropoietin from mammalian cell hosts containing the erythropoietin gene on recombinant plasmids has made available quantities of erythropoietin suitable for therapeutic applications. The polynucleotide and polypeptide sequences for several species of erythropoietin are known.

Currently, there are several recombinant human erythropoietin drug products on the market (e.g. Epogen®; epoetin alfa). In addition to epoetin alfa, there are other epoetins that have been developed (e.g., epoetin beta, delta, omega, zeta). In the context of the present invention, all forms of erythropoietins are meant to be encompassed when the term "erythropoietin" or "recombinant human erythropoietin" are used.

### Antibodies

Disclosed herein are antibodies, antibody fragments, antibody derivatives, antibody muteins, and antibody variants, that specifically bind to human erythropoietin.

The antibodies can comprise any constant region known in the art. The light chain constant region can be, for example, a kappa- or lambda-type light chain constant region, e.g., a human kappa- or lambda-type light chain constant region. The heavy chain constant region can be, for example, an alpha-, delta-, epsilon-, gamma-, or mu-type heavy chain constant regions, e.g., a human alpha-, delta-, epsilon-, gamma-, or mu-type heavy chain constant region. In one embodiment, the light or heavy chain constant region is a fragment, derivative, variant, or mutein of a naturally occurring constant region.

Disclosed herein is an antibody further comprising the constant light chain kappa or lambda domains or a fragment of these. Sequences of the light chain constant regions and polynucleotides encoding them well known in the art. Disclosed herein is an antibody further comprising a heavy chain constant domain, or a fragment thereof, such as the IgG1 or IgG2 heavy chain constant region, such sequences are well known in the art.

The antibodies of the present invention include those having a desired isotype (for example, IgA, IgG1, IgG2, IgG3, IgG4, IgM, IgE, and IgD) as well as Fab or F(ab')₂ fragments thereof. Moreover, if an IgG4 is desired, it may also be desired to introduce a point mutation in the hinge region as described in Bloom et al., 1997, Protein Science 6:407, to alleviate a tendency to form intra-H chain disulfide bonds that can lead to heterogeneity in the IgG4 antibodies.

The term "antibody" refers to an intact antibody, or an antigen binding fragment thereof, as described extensively in the Definitions section. An antibody may comprise a complete antibody molecule (including polyclonal, monoclonal, chimeric, humanized, or human versions having full length heavy and/or light chains), or comprise an antigen binding fragment thereof. Antibody fragments include F(ab')₂, Fab, Fab', Fv, Fc, and Fd fragments, and can be incorporated into single domain antibodies, single-chain antibodies, maxibodies, minibodies, intrabodies, diabodies, triabodies, tetrabodies, v-NAR and bis-scFv (see e.g., Hollinger and Hudson, 2005, Nature Biotechnology, 23, 9, 1126-1136). Also included are antibody polypeptides such as those disclosed in U. S. Patent No. 6,703,199, including fibronectin polypeptide monobodies. Other antibody polypeptides are disclosed in U.S. Patent Publication 2005/0238646, which are single-chain polypeptides. Disclosed herein are hybridomas capable of producing the antibodies of the invention, and methods of producing antibodies from hybridomas, as described further below.

Chimeric antibodies and humanized antibodies may be prepared by known techniques. Disclosed herein is a humanized monoclonal antibody comprising the variable domain of a murine antibody (or all or part of the antigen binding site thereof) and a constant domain derived from a human antibody. Alternatively, a humanized antibody fragment may comprise the antigen binding site of a murine monoclonal antibody and a variable domain fragment (lacking the antigen-binding site) derived from a human antibody. Procedures for the production of engineered monoclonal antibodies include those described in Riechmann et al., 1988, Nature 332:323, Liu et al., 1987, Proc. Nat. Acad. Sci. USA 84:3439, Larrick et al., 1989, Bio/Technology 7:934, and Winter et al., 1993, TIPS 14:139. The chimeric antibody may be a CDR grafted antibody. Techniques for humanizing antibodies are discussed in, e.g., U.S. Pat. Nos 5,869,619; 5,225,539; 5,821,337; 5,859,205; 6,881,557, Padlan et al., 1995, FASEB J. 9:133-39, Tamura et al., 2000, J. Immunol. 164:1432-41, Zhang, W., et al., Molecular Immunology. 42(12):1445-1451, 2005; Hwang W. et al., Methods. 36(1):35-42, 2005; Dall'Acqua WF, et al., Methods 36(1):43-60, 2005; and Clark, M., Immunology Today. 21(8):397-402, 2000.

An antibody may also be a fully human monoclonal antibody. Fully human monoclonal antibodies may be generated by any number of techniques with which those having ordinary skill in the art will be familiar. Such methods include, but are not limited to, Epstein Barr Virus (EBV) transformation of human peripheral blood cells (e.g., containing B lymphocytes), in vitro immunization of human B-cells, fusion of spleen cells from immunized transgenic mice carrying inserted human immunoglobulin genes, isolation from human immunoglobulin V region phage libraries, or other procedures as known in the art and based on the disclosure herein.

Procedures have been developed for generating human monoclonal antibodies in non-human animals. For example, mice in which one or more endogenous immunoglobulin genes have been inactivated by various means have been prepared. Human immunoglobulin genes have been introduced into the mice to replace the inactivated mouse genes. In this technique, elements of the human heavy and light chain locus are introduced into strains of mice derived from embryonic stem cell lines that contain targeted disruptions of the endogenous heavy chain and light chain loci (see also Bruggemann et al., Curr. Opin. Biotechnol. 8:455-58 (1997)). For example, human immunoglobulin transgenes may be mini-gene constructs, or transloci on yeast artificial chromosomes, which undergo B-cell-specific DNA rearrangement and hypermutation in the mouse lymphoid tissue.

Antibodies produced in the animal incorporate human immunoglobulin polypeptide chains encoded by the human genetic material introduced into the animal.A non-human animal, such as a transgenic mouse, is immunized with a suitable human erythropoietin immunogen.

Examples of techniques for production and use of transgenic animals for the production of human or partially human antibodies are described in U.S. Patents 5,814,318, 5,569,825, and 5,545,806, Davis et al., Production of human antibodies from transgenic mice in Lo, ed. Antibody Engineering: Methods and Protocols, Humana Press, NJ: 191-200 (2003), Kellermann et al., 2002, Curr Opin Biotechnol. 13:593-97, Russel et al., 2000, Infect Immun. 68:1820-26, Gallo et al., 2000, Eur J Immun. 30:534-40, Davis et al., 1999, Cancer Metastasis Rev. 18:421-25, Green, 1999, J Immunol Methods. 231:11-23, Jakobovits, 1998, Advanced Drug Delivery Reviews 31:33-42, Green et al., 1998, J Exp Med. 188:483-95, Jakobovits A, 1998, Exp. Opin. Invest. Drugs. 7:607-14, Tsuda et al., 1997, Genomics. 42:413-21, Mendez et al., 1997, Nat Genet. 15:146-56, Jakobovits, 1994, Curr Biol. 4:761-63, Arbones et al., 1994, Immunity. 1:247-60, Green et al., 1994, Nat Genet. 7:13-21, Jakobovits et al., 1993, Nature. 362:255-58, Jakobovits et al., 1993, Proc Natl Acad Sci USA. 90:2551-55. Chen, J., M. Trounstine, F. W. Alt, F. Young, C. Kurahara, J. Loring, D. Huszar. "Immunoglobulin gene rearrangement in B-cell deficient mice generated by targeted deletion of the JH locus." International Immunology 5 (1993): 647-656, Choi et al., 1993, Nature Genetics 4: 117-23, Fishwild et al., 1996, Nature Biotechnology 14: 845-51, Harding et al., 1995, Annals of the New York Academy of Sciences, Lonberg et al., 1994, Nature 368: 856-59, Lonberg, 1994, Transgenic Approaches to Human Monoclonal Antibodies in Handbook of Experimental Pharmacology 113: 49-101, Lonberg et al., 1995, Internal Review of Immunology 13: 65-93, Neuberger, 1996, Nature Biotechnology 14: 826, Taylor et al., 1992, Nucleic Acids Research 20: 6287-95, Taylor et al., 1994, International Immunology 6: 579-91, Tomizuka et al., 1997, Nature Genetics 16: 133-43, Tomizuka et al., 2000, Proceedings of the National Academy of Sciences USA 97: 722-27, Tuaillon et al., 1993, Proceedings of the National Academy of Sciences USA 90: 3720-24, and Tuaillon et al., 1994, Journal of Immunology 152: 2912-20.; Lonberg et al., Nature 368:856, 1994; Taylor et al., Int. Immun. 6:579, 1994; U.S. Patent No. 5,877,397; Bruggemann et al., 1997 Curr. Opin. Biotechnol. 8:455-58; Jakobovits et al., 1995 Ann. N. Y. Acad. Sci. 764:525-35. In addition, protocols involving the XenoMouse® (Abgenix, now Amgen, Inc.) are described, for example in U.S. 05/0118643 and WO 05/694879, WO 98/24838, WO 00/76310, and US Patent 7,064,244.

Lymphoid cells from the immunized transgenic mice are fused with myeloma cells for example to produce hybridomas. Myeloma cells for use in hybridoma-producing fusion procedures preferably are non-antibody-producing, have high fusion efficiency, and enzyme deficiencies that render them incapable of growing in certain selective media which support the growth of only the desired fused cells (hybridomas). Examples of suitable cell lines for use in such fusions include Sp-20, P3-X63/Ag8, P3-X63-Ag8.653, NS1/1.Ag 4 1, Sp210-Ag14, FO, NSO/U, MPC-11, MPC11-X45-GTG 1.7 and S194/5XX0 Bul; examples of cell lines used in rat fusions include R210.RCY3, Y3-Ag 1.2.3, IR983F and 4B210. Other cell lines useful for cell fusions are U-266, GM1500-GRG2, LICR-LON-HMy2 and UC729-6.

The lymphoid (e.g., spleen) cells and the myeloma cells may be combined for a few minutes with a membrane fusion-promoting agent, such as polyethylene glycol or a nonionic detergent, and then plated at low density on a selective medium that supports the growth of hybridoma cells but not unfused myeloma cells. One selection media is HAT (hypoxanthine, aminopterin, thymidine). After a sufficient time, usually about one to two weeks, colonies of cells are observed. Single colonies are isolated, and antibodies produced by the cells may be tested for binding activity to human erythropoietin using any one of a variety of immunoassays known in the art and described herein. The hybridomas are cloned (e.g., by limited dilution cloning or by soft agar plaque isolation) and positive clones that produce an antibody specific to human erythropoietin are selected and cultured. The monoclonal antibodies from the hybridoma cultures may be isolated from the supernatants of hybridoma cultures. Thus, disclosed herein are hybridomas that comprise polynucleotides encoding the antibodies of the invention in the chromosomes of the cell. These hybridomas can be cultured according to methods described herein and known in the art.

Another method for generating human antibodies includes immortalizing human peripheral blood cells by EBV transformation. See, e.g., U.S. Patent No. 4,464,456. Such an immortalized B-cell line (or lymphoblastoid cell line) producing a monoclonal antibody that specifically binds to human erythropoietin can be identified by immunodetection methods as provided herein, for example, an ELISA, and then isolated by standard cloning techniques. The stability of the lymphoblastoid cell line producing an anti-human erythropoietin antibody may be improved by fusing the transformed cell line with a murine myeloma to produce a mouse-human hybrid cell line according to methods known in the art (see, e.g., Glasky et al., Hybridoma 8:377-89 (1989)). Still another method to generate human monoclonal antibodies is in vitro immunization, which includes priming human splenic B-cells with human erythropoietin, followed by fusion of primed B-cells with a heterohybrid fusion partner. See, e.g., Boerner et al., 1991 J. Immunol. 147:86-95.

A B-cell that is producing an anti- human erythropoietin antibody may be selected and the light chain and heavy chain variable regions cloned from the B-cell according to molecular biology techniques known in the art (WO 92/02551; U.S. patent 5,627,052; Babcook et al., Proc. Natl. Acad. Sci. USA 93:7843-48 (1996)) and described herein. B-cells from an immunized animal may be isolated from the spleen, lymph node, or peripheral blood sample by selecting a cell that is producing an antibody that specifically binds to human erythropoietin. B-cells may also be isolated from humans, for example, from a peripheral blood sample. Methods for detecting single B-cells that are producing an antibody with the desired specificity are well known in the art, for example, by plaque formation, fluorescence-activated cell sorting, in vitro stimulation followed by detection of specific antibody, and the like. Methods for selection of specific antibody-producing B-cells include, for example, preparing a single cell suspension of B-cells in soft agar that contains human erythropoietin. Binding of the specific antibody produced by the B-cell to the antigen results in the formation of a complex, which may be visible as an immunoprecipitate. After the B-cells producing the desired antibody are selected, the specific antibody genes may be cloned by isolating and amplifying DNA or mRNA according to methods known in the art and described herein.

An additional method for obtaining antibodies is by phage display. See, e.g., Winter et al., 1994 Annu. Rev. Immunol. 12:433-55; Burton et al., 1994 Adv. Immunol. 57:191-280. Human or murine immunoglobulin variable region gene combinatorial libraries may be created in phage vectors that can be screened to select Ig fragments (Fab, Fv, sFv, or multimers thereof) that bind specifically to TGF-beta binding protein or variant or fragment thereof. See, e.g., U.S. Patent No. 5,223,409; Huse et al., 1989 Science 246:1275-81; Sastry et al., Proc. Natl. Acad. Sci. USA 86:5728-32 (1989); Alting-Mees et al., Strategies in Molecular Biology 3:1-9 (1990); Kang et al., 1991 Proc. Natl. Acad. Sci. USA 88:4363-66; Hoogenboom et al., 1992 J. Molec. Biol. 227:381-388; Schlebusch et al., 1997 Hybridoma 16:47-52 and references cited therein. For example, a library containing a plurality of polynucleotide sequences encoding Ig variable region fragments may be inserted into the genome of a filamentous bacteriophage, such as M13 or a variant thereof, in frame with the sequence encoding a phage coat protein. A fusion protein may be a fusion of the coat protein with the light chain variable region domain and/or with the heavy chain variable region domain. Immunoglobulin Fab fragments may also be displayed on a phage particle (see, e.g., U.S. Patent No. 5,698,426).

Heavy and light chain immunoglobulin cDNA expression libraries may also be prepared in lambda phage, for example, using λlmmunoZap™(H) and λImmunoZap™(L) vectors (Stratagene, La Jolla, California). Briefly, mRNA is isolated from a B-cell population, and used to create heavy and light chain immunoglobulin cDNA expression libraries in the λImmunoZap(H) and λImmunoZap(L) vectors. These vectors may be screened individually or co-expressed to form Fab fragments or antibodies (see Huse et al., supra; see also Sastry et al., supra). Positive plaques may subsequently be converted to a non-lytic plasmid that allows high level expression of monoclonal antibody fragments from E. coli.

In a hybridoma, the variable regions of a gene expressing a monoclonal antibody of interest may be amplified using nucleotide primers. These primers may be synthesized by one of ordinary skill in the art, or may be purchased from commercially available sources. (See, e.g., Stratagene (La Jolla, California), which sells primers for mouse and human variable regions including, among others, primers for V_{Ha}, V_{Hb}, V_{Hc}, V_{Hd}, C_{H1}, V_{L} and CL regions.) These primers may be used to amplify heavy or light chain variable regions, which may then be inserted into vectors such as ImmunoZAP™H or ImmunoZAP™L (Stratagene), respectively. These vectors may then be introduced into E. coli, yeast, or mammalian-based systems for expression. Large amounts of a single-chain protein containing a fusion of the V_{H} and V_{L} domains may be produced using these methods (see Bird et al., Science 242:423-426, 1988).

Once cells producing antibodies according have been obtained using any of the above-described immunization and other techniques, the specific antibody genes may be cloned by isolating and amplifying DNA or mRNA therefrom according to standard procedures as described herein. The antibodies produced therefrom may be sequenced and the CDRs identified and the DNA coding for the CDRs may be manipulated as described previously to generate other antibodies according to the invention.

Antibodies may be generated by first identifying antibodies that bind to cells expressing human erythropoietin and/or compete for binding with the antibodies described in this application.

It will be understood by one skilled in the art that some proteins, such as antibodies, may undergo a variety of posttranslational modifications. The type and extent of these modifications often depends on the host cell line used to express the protein as well as the culture conditions. Such modifications may include variations in glycosylation, methionine oxidation, diketopiperizine formation, aspartate isomerization and asparagine deamidation. A frequent modification is the loss of a carboxy-terminal basic residue (such as lysine or arginine) due to the action of carboxypeptidases (as described in Harris, R.J. Journal of Chromatography 705:129-134, 1995).

An alternative method for production of a murine monoclonal antibody is to inject the hybridoma cells into the peritoneal cavity of a syngeneic mouse, for example, a mouse that has been treated (e.g., pristane-primed) to promote formation of ascites fluid containing the monoclonal antibody. Monoclonal antibodies can be isolated and purified by a variety of well-established techniques. Such isolation techniques include affinity chromatography with Protein-A Sepharose, size-exclusion chromatography, and ion-exchange chromatography (see, for example, Coligan at pages 2.7.1-2.7.12 and pages 2.9.1-2.9.3; Baines et al., "Purification of Immunoglobulin G (IgG)," in Methods in Molecular Biology, Vol. 10, pages 79-104 (The Humana Press, Inc. 1992)). Monoclonal antibodies may be purified by affinity chromatography using an appropriate ligand selected based on particular properties of the antibody (e.g., heavy or light chain isotype, binding specificity, etc.). Examples of a suitable ligand, immobilized on a solid support, include Protein A, Protein G, an anticonstant region (light chain or heavy chain) antibody, an anti-idiotype antibody, and a TGF-beta binding protein, or fragment or variant thereof.

Molecular evolution of the complementarity determining regions (CDRs) in the center of the antibody binding site also has been used to isolate antibodies with increased affinity, for example, antibodies having increased affinity for c-erbB-2, as described by Schier et al., 1996, J. Mol. Biol. 263:551. Accordingly, such techniques are useful in preparing antibodies to human erythropoietin.

Antibodies directed against human erythropoietin can be used, for example, in assays to detect the presence of human erythropoietin, either in vitro or in vivo.

Antibodies also may be prepared by any of a number of conventional techniques. For example, they may be purified from cells that naturally express them (e.g., an antibody can be purified from a hybridoma that produces it), or produced in recombinant expression systems, using any technique known in the art. See, for example, Monoclonal Antibodies, Hybridomas: A New Dimension in Biological Analyses, Kennet et al. (eds.), Plenum Press, New York (1980); and Antibodies: A Laboratory Manual, Harlow and Land (eds.), Cold Spring Harbor Laboratory Press, Cold Spring Harbor, NY, (1988). This is discussed in the nucleic acid section below.

Where it is desired to improve the affinity of antibodies according to the invention containing one or more of the above-mentioned CDRs can be obtained by a number of affinity maturation protocols including maintaining the CDRs (Yang et al., J. Mol. Biol., 254, 392-403, 1995), chain shuffling (Marks et al., Bio/Technology, 10, 779-783, 1992), use of mutation strains of E. coli. (Low et al., J. Mol. Biol., 250, 350-368, 1996), DNA shuffling (Patten et al., Curr. Opin. Biotechnol., 8, 724-733, 1997), phage display (Thompson et al., J. Mol. Biol., 256, 7-88, 1996) and additional PCR techniques (Crameri, et al., Nature, 391, 288-291, 1998). All of these methods of affinity maturation are discussed by Vaughan et al. (Nature Biotechnology, 16, 535-539, 1998).

### Conjugates

Agents can be conjugated to the discslosed antibodies. Such antibodies can be useful for detecting the presence or absence of recombinant proteins, such as, but not limited to, erythropoietin. These conjugates can be generated as fusion proteins. Numerous proteins, enzymes, florochromes, isotopes or other detectable labels can optionally be conjugated to the antibodies. Antibodies may optionally be covalently or non-covalently linked to such a detectable label.

Detectable labels suitable for such use include, but are not limited to, various enzymes, such as, horseradish peroxidase (HRP), alkaline phosphatase, beta-galactosidase, or acetylcholinesterase; prosthetic groups, such as, but not limited to, streptavidin/biotin and avidin/biotin; fluorescent materials, such as, but not limited to, umbelliferone, fluorescein, fluorescein isothiocynate, rhodamine, dichlorotriazinylamine fluorescein, dansyl chloride or phycoerythrin; luminescent materials, such as, but not limited to, luminol; bioluminescent materials, such as but not limited to, luciferase, luciferin, and acquorin; radioactive materials, such as, but not limited to, iodine (¹³¹I, ¹²⁵I, ¹²³I, and ¹²¹I), carbon (¹³¹I, sulfur (³⁵S), tritium (³H), indium (¹¹⁵In, ¹¹³In, ¹¹²In, and ¹¹¹In), technetium (⁹⁹Tc), thallium (²⁰¹Ti), (⁶⁸Ga, ⁶⁷Ga), palladium (¹⁰³Pd), molybdenum (⁹⁹Mo), xenon (¹³³Xe), fluorine (¹⁸F), ¹⁵³Sm, ¹⁷⁷Lu, ¹⁵⁹Gd, ¹⁴⁹Pm, ¹⁴⁰La, ¹⁷⁵Yb, ¹⁶⁶Ho, ⁹⁰Y, ⁴⁷Sc, ¹⁸⁶Re, ¹⁸⁸Re, ¹⁴²Pr, ¹⁰⁵Rh, ⁹⁷Ru, ⁶⁸Ge, ⁵⁷Co, ⁶⁵Zn, ⁸⁵Sr, ³²P, ¹⁵³Gd, ¹⁶⁹Yb, ⁵¹Cr, ⁵⁴Mn, ⁷⁵Se, ¹¹³Sn, and ¹¹⁷Sn; and positron emitting metals using various positron emission tomographies, and nonradioactive paramagnetic metal ions.

Covalent modifications of the antibodies are contemplated. They may be made by chemical synthesis or by enzymatic or chemical cleavage of the antibody, if applicable. Other types of covalent modifications of the anti-human erythropoietin antibody are introduced into the molecule by reacting targeted amino acid residues of the antibody with an organic derivatizing agent that is capable of reacting with selected side chains or the N- or C-terminal residues.

Cysteinyl residues most commonly are reacted with alpha-haloacetates (and corresponding amines), such as chloroacetic acid or chloroacetamide, to give carboxymethyl or carboxyamidomethyl derivatives. Similarly, iodo-reagents may also be used. Cysteinyl residues also are derivatized by reaction with bromotrifluoroacetone, alpha-bromo-beta-(5-imidozoyl)propionic acid, chloroacetyl phosphate, N-alkylmaleimides, 3-nitro-2-pyridyl disulfide, methyl 2-pyridyl disulfide, p-chloromercuribenzoate, 2-chloromercuri-4-nitrophenol, or chloro-7-nitrobenzo-2-oxa-1,3-diazole.

Histidyl residues are derivatized by reaction with diethylpyrocarbonate at pH 5.5-7.0 because this agent is relatively specific for the histidyl side chain. Para-bromophenacyl bromide also is useful; the reaction is preferably performed in 0.1 M sodium cacodylate at pH 6.0.

Lysyl and amino-terminal residues are reacted with succinic or other carboxylic acid anhydrides. Derivatization with these agents has the effect of reversing the charge of the lysinyl residues. Other suitable reagents for derivatizing .alpha.-amino-containing residues and/or e-amino-containing residues include imidoesters such as methyl picolinimidate, pyridoxal phosphate, pyridoxal, chloroborohydride, trinitrobenzenesulfonic acid, 0-methylisourea, 2,4-pentanedione, and transaminase-catalyzed reaction with glyoxylate.

Arginyl residues are modified by reaction with one or several conventional reagents, among them phenylglyoxal, 2,3-butanedione, 1,2-cyclohexanedione, and ninhydrin. Derivatization of arginyl residues generally requires that the reaction be performed in alkaline conditions because of the high pKa of the guanidine functional group. Furthermore, these reagents may react with the epsilon-amino groups of lysine as well as the arginine epsilon-amino group.

The specific modification of tyrosyl residues may be made, with particular interest in introducing spectral labels into tyrosyl residues by reaction with aromatic diazonium compounds or tetranitromethane. Most commonly, N-acetylimidizole and tetranitromethane are used to form O-acetyl tyrosyl species and 3-nitro derivatives, respectively. Tyrosyl residues are iodinated using I¹²⁵ or I¹³¹ to prepare labeled proteins for use in radioimmunoassay.

Carboxyl side groups (aspartyl or glutamyl) are selectively modified by reaction with carbodiimides (R-N==C==N-R'), where R and R' are different alkyl groups, such as 1-cyclohexyl-3-(2-morpholinyl-4-ethyl)carbodiimide or 1-ethyl-3-(4-azonia-4,4-dimethylpentyl)carbodiimide. Furthermore, aspartyl and glutamyl residues are converted to asparaginyl and glutaminyl residues by reaction with ammonium ions.

Glutaminyl and asparaginyl residues are frequently deamidated to the corresponding glutamyl and aspartyl residues, respectively. These residues are deamidated under neutral or basic conditions. The deamidated form of these residues falls within the scope of this invention.

Other modifications include hydroxylation of proline and lysine, phosphorylation of hydroxyl groups of seryl or threonyl residues, methylation of the .alpha.-amino groups of lysine, arginine, and histidine side chains (T. E. Creighton, Proteins: Structure and Molecular Properties, W.H. Freeman & Co., San Francisco, pp. 79-86 (1983)), acetylation of the N-terminal amine, and amidation of any C-terminal carboxyl group.

Another type of covalent modification involves chemically or enzymatically coupling glycosides to the antibody. These procedures are advantageous in that they do not require production of the antibody in a host cell that has glycosylation capabilities for N- or O-linked glycosylation. Depending on the coupling mode used, the sugar(s) may be attached to (a) arginine and histidine, (b) free carboxyl groups, (c) free sulfhydryl groups such as those of cysteine, (d) free hydroxyl groups such as those of serine, threonine, or hydroxyproline, (e) aromatic residues such as those of phenylalanine, tyrosine, or tryptophan, or (f) the amide group of glutamine. These methods are described in WO 87/05330 published 11 Sep. 1987, and in Aplin and Wriston, CRC Crit. Rev. Biochem., pp. 259-306 (1981).

### Assays

The invention can be used to assay for the presence or absence of recombinant proteins, such as human erythropoietin, in a sample using classical immunohistological methods as described herein or as known to those of skill in the art (e.g., see Jalkanen et al., 1985, J. Cell. Biol. 101:976-985; and Jalkanen et al., 1987, J. Cell. Biol. 105:3087-3096). Other nonlimiting examples of immunological-based methods useful for detecting protein gene expression include immunoassays. Suitable antibody assay labels are known in the art and nonlimiting examples are described herein. The anti-human erythropoietin antibodies may be labeled, as described herein.

Detecting the presence of an antibody-antigen complex comprising an antibody that selectively binds to a protein, such as erythropoietin, is also disclosed. It is envisioned that any detection system can be used to practice aspects of this disclosed immuno-based method, with the provision that the signal to noise ratio can distinguish to a statistically significant degree the signal from the antibody-antigen complex from the background signal. Non-limiting examples of immuno-based detection systems include immunoblot analysis, like Western blotting and dot-blotting, immunoprecipitation analysis, enzyme-linked immunosorbent analysis (ELISA), and sandwich ELISA. The detection of the signal can be achieved using autoradiography with imaging or phosphorimaging (AU), chemiluminescense (CL), electrochemiluminescence (ECL), bioluminescence (BL), fluorescence, resonance energy transfer, plane polarization, colormetric, or flow cytometry (FC). Descriptions of immuno-based detection systems are disclosed in, e.g., Michael M. Rauhut, Chemiluminescence, In Kirk-Othmer Concise Encyclopedia of Chemical Technology (Ed. Grayson, 3rd ed, John Wiley and Sons, 1985); A. W. Knight, A Review of Recent Trends in Analytical Applications of Electrogenerated Chemiluminescence, Trends Anal. Chem. 18(1): 47-62 (1999); K. A. Fahnrich, et al., Recent Applications of Electrogenerated Chemiluminescence in Chemical Analysis, Talanta 54(4): 531-559 (2001); Commonly Used Techniques in Molecular Cloning, pp. A8.1-A8-55 (Sambrook & Russell, eds., Molecular Cloning A Laboratory Manual, Vol. 3, 3.sup.rd ed. 2001); Detection Systems, pp. A9.1-A9-49 (Sambrook & Russell, eds., Molecular Cloning A Laboratory Manual, Vol. 3, 3.sup.rd ed. 2001); Electrogenerated Chemiluminescence, (Ed. Allen J. Bard, Marcel Dekker, Inc., 2004).

A sandwich ELISA (or sandwich immunoassay) is a method based on two antibodies, which bind to different epitopes on the antigen. A capture antibody having a high binding specificity for the antigen of interest, is bound to a solid surface. The antigen is then added followed by addition of a second antibody referred to as the detection antibody. The detection antibody binds the antigen to a different epitope than the capture antibody. The antigen is therefore 'sandwiched' between the two antibodies. The antibody binding affinity for the antigen is usually the main determinant of immunoassay sensitivity. As the antigen concentration increases the amount of detection antibody increases leading to a higher measured response. To quantify the extent of binding different reporter systems can be used, such as, e.g., an enzyme attached to the secondary antibody and a reporter substrate where the enzymatic reaction forms a readout as the detection signal. The signal generated is proportional to the amount of target antigen present in the sample. The reporter substrate used to measure the binding event determines the detection mode. A spectrophotometric plate reader is used for colorimetric detection. Chemiluminescent and electrochemiluminescence substrates have been developed which further amplify the signal and can be read on a luminescent reader. The reporter can also be a fluorescent readout where the enzyme step of the assay is replaced with a fluorophore and the readout is then measured using a fluorescent reader. Reagents and protocols necessary to perform an ECL sandwich ELISA are commercially available, including, but not limited to, MSD sandwich ELISA-ECL detection platform (Meso Scale Discovery, Gaithersburg, Md.).

Disclosed herein is a method of detecting the presence of human erythropoietin in a sample. The method may comprise contacting the sample with an anti-human erythropoietin antibody under conditions permissive for binding of the anti-human erythropoietin antibody to human erythropoietin, and detecting whether a complex is formed between the anti-human erythropoietin antibody and human erythropoietin.

Anti-human erythropoietin antibodies may be immobilized on an insoluble matrix. Immobilization entails separating the anti-human erythropoietin antibody from any human erythropoietin that remains free in solution. This conventionally is accomplished by either insolubilizing the anti-human erythropoietin antibody before the assay procedure, as by adsorption to a water-insoluble matrix or surface (Bennich et al., U.S. Pat. No. 3,720,760), or by covalent coupling (for example, using glutaraldehyde cross-linking), or by insolubilizing the anti-human erythropoietin antibody after formation of a complex between the anti-human erythropoietin antibody and human erythropoietin, e.g., by immunoprecipitation.

Disclosed herein is an immuno-based method of detecting recombinant proteins, such as erythropoietin. The immuno-based methods disclosed in the present specification can be evaluated by several parameters including, e.g., accuracy, precision, limit of detection (LOD), limits of quantitation (LOQ), linear range, specificity, selectivity, linearity, ruggedness, and system suitability. The accuracy of a method is the measure of exactness of an analytical method, or the closeness of agreement between the measured value and the value that is accepted as a conventional true value or an accepted reference value. The precision of a method is the degree of agreement among individual test results, when the procedure is applied repeatedly to multiple samplings of a homogeneous sample. As such, precision evaluates 1) within assay variability; 2) within-day variability (repeatability); and 3) between-day variability (intermediate precision); and 4) between-lab variability (reproducibility). Coefficient of variation (CV %) is a quantitative measure of precision expressed relative to the observed or theoretical mean value.

An immuno-based method disclosed in the present specification must be able to detect, over background, the presence of an antibody-antigen complex. The limit of detection (LOD) of a method refers to the concentration of analyte which gives rise to a signal that is significantly different from the negative control or blank and represents the lowest concentration of analyte that can be distinguished from background. Thus, the immuno-based method disclosed in the present specification can detect the LOD at an amount that is significantly different from a negative control or blank.

The limits of quantitation (LOQ) are the lowest and the highest concentrations of analyte in a sample or specimen that can be measured with an acceptable level of accuracy and precision. The lower limit of quantitation refers to the lowest dose that a detection method can measure consistently from the background. The upper limit of quantitation is the highest dose that a detection method can measure consistently before saturation of the signal occurs. The linear range of the method is the area between the lower and the upper limits of quantitation. The linear range is calculated by subtracting lower limit of quantitation from the upper limit of quantitation. Thus, in an embodiment, the immuno-based method disclosed in the present specification can detect the LOQ at an amount that is significantly different from a negative control or blank.

Disclosed herein is an antibody linked to a solid phase support. As used herein, the term "solid-phase support" is synonymous with "solid phase" and refers to any matrix that can be used for immobilizing an antibody disclosed in the present specification. Non-limiting examples of solid phase supports include, e.g., a tube; a plate; a column; pins or "dipsticks"; a magnetic particle, a bead or other spherical or fibrous chromatographic media, such as, e.g., agarose, sepharose, silica and plastic; and sheets or membranes, such as, e.g., nitrocellulose and polyvinylidene fluoride (PVDF). The solid phase support can be constructed using a wide variety of materials such as, e.g., glass, carbon, polystyrene, polyvinylchloride, polypropylene, polyethylene, dextran, nylon, diazocellulose, or starch. The solid phase support selected can have a physical property that renders it readily separable from soluble or unbound material and generally allows unbound materials, such as, e.g., excess reagents, reaction by-products, or solvents, to be separated or otherwise removed (by, e.g., washing, filtration, centrifugation, etc.) from solid phase support-bound assay component. Non-limiting examples of how to make and use a solid phase supports are described in, e.g., Molecular Cloning, A Laboratory Manual, supra, (2001); and Current Protocols in Molecular Biology, supra, (2004).

The invention having been described, the following examples are offered by way of illustration, and not limitation.

### EXAMPLES

### Example 1:

### SUMMARY OF EXPERIMENTS

Rabbit anti-EPO HRP were diluted to 10 ng/mL using lyophilization buffer (LBT: 1% BSA, 10mM Potassium phosphate, 4% Mannitol, 2% Sucrose, 0.05% polysorbate 20, pH 7.0) and aliquoted 2 mL in a 5 cc vial before lyophilization. This lyophilized conjugate (10 ng/mL) was reconstituted with 2 mL of water and use for one sample analysis. The lyophilized conjugate can be stored at room temperature for up to 1 year and can be tested for stability of conjugate by ELISA at 3 month, 6 month and 1 yr if it is required for stability. Epogen bulk was formulated in same formulation buffer (LBT: 1% BSA, 10mM Potassium phosphate, 4% Mannitol, 2% Sucrose, 0.05% polysorbate 20, pH 7.0) to be 2000 U/mL in 5 cc vial.

### MATERIALS AND EQUIPMENT

### Materials

Rb Anti-EPO HRP: 0.63mg/mL (630 ng/uL), 0.67 mL/vial
EPO: RB-EPO FPB, 3.84 mg/mL

### Formulation buffer:

### 1x PBS: Phosphate buffered Saline solution:

8g NaCl, 0.2g KCl, 1.44g Na2HPO4, 0.24gKH2PO4 in 800 mL of distilled water and adjust the pH to 7.4 with HCl and add water to make 1 liter

### Sample dilution buffer (BSAT):

1% Bovine Serum Albumin in 1x PBS with 0.05% polysorbate 20

### Lyophilization buffer (LBT):

1% BSA, 10mM Potassium phosphate, 4% Mannitol, 2% Sucrose, 0.05% polysorbate 20, pH 7.0

### Equipment

B2 Lyophilization instrument
5 cc vials
Lyophilization stopper

### TEST PROCEDURES

### Sample Preparation for conjugate:

Each sample diluted according to the sample table below (Table 1) to make 10 ng/mL with Lyophilization buffer (LBT). Pipeted 2 mL of sample in a 5cc vial. Samples were lyophilized according to the procedure. After Lyophilization, samples were stored in room temperature. Reconstituted the sample with 2 mL water per each plate before use

**Table 1. Sample dilution scheme for lyophilization formulation of conjugate**

| | Rb Anti-EPO HRP |
|---|---|
| Starting Concentration (SS) | 0.63mg/mL (630 ng/uL) |
| A | 5 uL(SS) + 495 uL (LBT) |
| B | 0.5 mL (A) + 314.5 mL (LBT) |
| Final concentration | 10 ng/mL |
| Final volume | 315 mL |
| Final # vial | 150 (2 mL in 5 cc vial) |

### Sample Preparation for EPO:

Diluted each sample according to the sample table below (Table 2) to make 16.8 ug/mL (2019U/mL) with Lyophilization buffer (LBT), Pipeted 2 mL of sample in a 5cc vial.

Samples were lyophilized according to the procedure. After Lyophilization, samples were stored in room temperature. Reconstituted the sample with 2 mL water per each plate before use.

**Table 2. Sample dilution scheme for lyophilization formulation of EPO**

| | EPO |
|---|---|
| Starting Concentration (SS) | 3840 ug/mL |
| EPO bulk amount (mL) | 1.76 mL |
| Buffer (mL) | 400 mL |
| Final concentration | 16.8 ug/mL (2019 U/mL) |
| Final volume | 400 mL |
| Final # vial | 200 (2 mL per 5cc vial) |

### Placebo Preparation

Pipeted 2 mL of LBT in a 5 cc vial and lyophilize with sample. Made 20 vials.

### Lyophilization procedure

Sample vials were loaded on 'pre-chilled" (4°C) shelves of a lyophilizer. Samples were subjected for three steps of freezing, primary drying and secondary drying. First the samples were held for 30 minutes at 4 °C and then cooled down from 4 °C to -45 °C over 123 minutes and held at -45 °C for 180 minutes. The shelf temperature was raised to -12 °C over 150 minutes and held at -12 °C for 240 minutes. Then the temperature was lowered to -45 °C again over 150 min and held at -45C for 120 minutes. For primary drying, the chamber vacuum was lowered to 120 mTorr and cooled the condenser to less than -50 °C. Subsequently, the shelf temperature was raised to -10 °C and held for 25 hours keeping the chamber vacuum at 120 mTorr. For secondary drying, the shelf temperature was raised to 25 °C over 234 min while lowering chamber pressure to 100 mTorr. After that the shelf temperature was held 25 °C for 11 hours while keeping the chamber pressure at 100 mTorr. After completion of the secondary drying step, the vials were stoppered inside the drying chamber at a chamber pressure of 9/10 of one atmosphere.

### Media Preparation

| Additional Information | Comments/Added Steps |
|---|---|
| Filter @ 0.22 uM | 1% Bovine Serum albumin, 10 mM potassium phosphate, 4% Mannitol, 2% sucrose, 0.05% polysorbate 20, pH 7.0 |

### ELISA assay for EPO

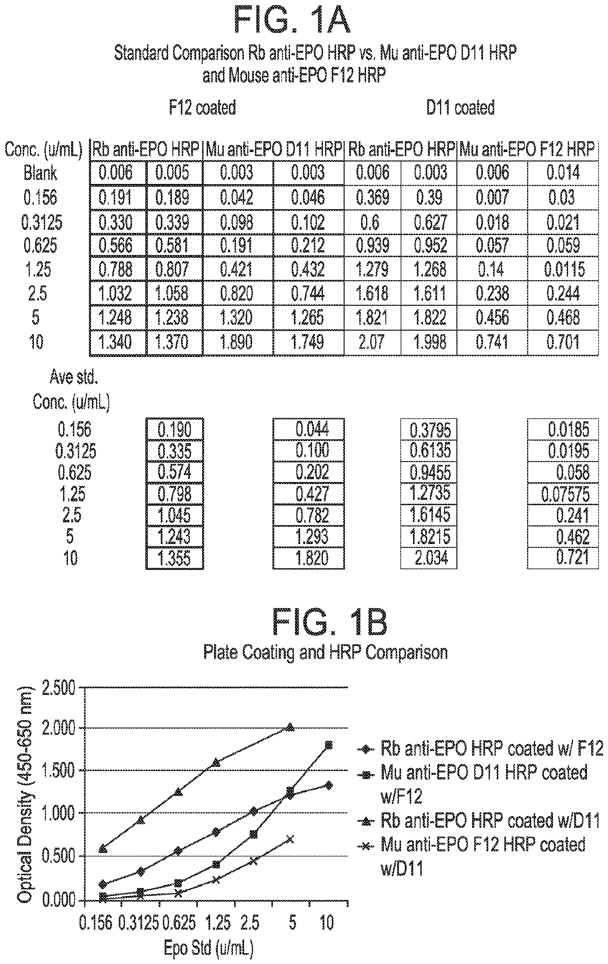

Plate was coated with Mu anti-EPO F12 vs. Mu anti EPO D11 at 2.5 ug/mL. EPO standards were incubated. Erythropoietin bound to the immobilized antibody on the plate. After removing excess standard, wells were incubated with Rb anti-EPO HRP at concentration of 10 ng/mL. During this incubation period the conjugate bound to the immobilized EPO. Excess conjugate was removed by washing. Chromagen was added and was oxidized by the enzyme reaction to form a blue colored complex then the reaction was stopped by the addition of acid 1M Phosporic acid which turns blue to yellow. Plate was read at 450-650 nm. The results of this experiment are summarized in Figure 1.

### Stability Test

Samples were stored at 25°C and 40°C to be tested for up to 1 year for stability. These samples can be tested at at least time zero, 3 mo, 6 mo and lyear by ELISA.

The present invention is not to be limited in scope by the specific embodiments described herein, which are intended as single illustrations of individual aspects of the invention, and functionally equivalent methods and components are invention. Indeed, various modifications of the invention, in addition to those shown and described herein will become apparent to those skilled in the art from the foregoing description and accompanying drawings. Such modifications are intended to fall within the scope of the appended claims.

## Claims

1. A stable, solid protein formulation comprising bovine serum albumin (BSA), potassium phosphate, mannitol, sucrose, and polysorbate 20, wherein said protein is darbepoetin.

2. A lyophilization buffer comprising bovine serum albumin (BSA), potassium phosphate, mannitol, sucrose, and polysorbate 20, optionally wherein the buffer further comprises darbepoetin.

3. The formulation of claim 1 or the buffer of claim 2, wherein the concentration of BSA is between 0.1% and 10%.

4. The formulation of claim 1 or the buffer of claim 2, wherein the concentration of potassium phosphate is between 1mM and 100mM.

5. The formulation of claim 1 or the buffer of claim 2, wherein the concentration of mannitol is between 1% and 10%.

6. The formulation of claim 1 or the buffer of claim 2, wherein the concentration of sucrose is between 1% and 10%.

7. The formulation of claim 1 or the buffer of claim 2, wherein the concentration of polysorbate 20 is between 0.005% and 0.5%.

8. The formulation of claim 1 or the buffer of claim 2, wherein the pH is between 6.0 and 8.0.

9. The formulation of claim 1 or the buffer of claim 2, wherein the concentration of protein is between 1 ng/ml and 100 ng/ml.

10. The formulation or buffer of any of claims 1-9, wherein said formulation is lyophilized.

11. The formulation or buffer of claim 10, wherein said formulation is reconstituted with water.

12. A method of preparing a stable, solid protein formulation, said method comprising:
a) diluting said protein with the lyophilization buffer of any of claims 2 to 11; and
b) lyophilizing said diluted protein

## Patentansprüche

1. Stabile, feste Proteinformulierung, umfassend Rinderserumalbumin (BSA), Kaliumphosphat, Mannitol, Saccharose und Polysorbat 20, wobei es sich bei dem Protein um Darbepoetin handelt.

2. Lyophilisierungspuffer, umfassend Rinderserumalbumin (BSA), Kaliumphosphat, Mannitol, Saccharose und Polysorbat 20, gegebenenfalls wobei der Puffer Darbepoetin umfasst.

3. Formulierung nach Anspruch 1 oder Puffer nach Anspruch 2, wobei die Konzentration von BSA zwischen 0,1 % und 10 % liegt.

4. Formulierung nach Anspruch 1 oder Puffer nach Anspruch 2, wobei die Konzentration von Kaliumphosphat zwischen 1 mM und 100 mM liegt.

5. Formulierung nach Anspruch 1 oder Puffer nach Anspruch 2, wobei die Konzentration von Mannitol zwischen 1 % und 10 % liegt.

6. Formulierung nach Anspruch 1 oder Puffer nach Anspruch 2, wobei die Konzentration von Saccharose zwischen 1 % und 10 % liegt.

7. Formulierung nach Anspruch 1 oder Puffer nach Anspruch 2, wobei die Konzentration von Polysorbat 20 zwischen 0,005 % und 0,5 % liegt.

8. Formulierung nach Anspruch 1 oder Puffer nach Anspruch 2, wobei der pH-Wert zwischen 6,0 und 8,0 liegt.

9. Formulierung nach Anspruch 1 oder Puffer nach Anspruch 2, wobei die Konzentration von Protein zwischen 1 ng/ml und 100 ng/ml liegt.

10. Formulierung oder Puffer nach einem der Ansprüche 1-9, wobei die Formulierung lyophilisiert ist.

11. Formulierung oder Puffer nach Anspruch 10, wobei die Formulierung mit Wasser rekonstituiert ist.

12. Verfahren zur Herstellung einer stabilen, festen Proteinformulierung, wobei das Verfahren Folgendes umfasst:
a) Verdünnen des Proteins mit dem Lyophilisierungspuffer nach einem der Ansprüche 2 bis 11 und
b) Lyophilisieren des verdünnten Proteins.

## Revendications

1. Formulation protéique solide, stable comprenant de l'albumine de sérum bovin (ABS), du phosphate de potassium, du mannitol, du saccharose, et un polysorbate 20, ladite protéine étant la darbépoétine.

2. Tampon de lyophilisation comprenant de l'albumine de sérum bovin (ABS), du phosphate de potassium, du mannitol, du saccharose, et un polysorbate 20, éventuellement le tampon comprenant en outre de la darbépoétine.

3. Formulation selon la revendication 1 ou tampon selon la revendication 2, la concentration de l'ABS étant comprise entre 0,1 % et 10 %.

4. Formulation selon la revendication 1 ou tampon selon la revendication 2, la concentration du phosphate de potassium étant comprise entre 1 mM et 100 mM.

5. Formulation selon la revendication 1 ou tampon selon la revendication 2, la concentration du mannitol étant comprise entre 1 % et 10 %.

6. Formulation selon la revendication 1 ou tampon selon la revendication 2, la concentration du saccharose étant comprise entre 1 % et 10 %.

7. Formulation selon la revendication 1 ou tampon selon la revendication 2, la concentration du polysorbate 20 étant comprise entre 0,005 % et 0,5 %.

8. Formulation selon la revendication 1 ou tampon selon la revendication 2, le pH étant compris entre 6,0 et 8,0.

9. Formulation selon la revendication 1 ou tampon selon la revendication 2, la concentration de protéine étant comprise entre 1 ng/ml et 100 ng/ml.

10. Formulation ou tampon selon l'une quelconque des revendications 1 à 9, ladite formulation étant lyophilisée.

11. Formulation ou tampon selon la revendication 10, ladite formulation étant reconstituée avec de l'eau.

12. Procédé de préparation d'une formulation protéique solide, stable, ledit procédé comprenant :
a) la dilution de ladite protéine avec le tampon de lyophilisation selon l'une quelconque des revendications 2 à 11 ; et
b) la lyophilisation de ladite protéine diluée.
